# EUROPEAN PATENT APPLICATION

(11) **EP 2 572 631 A2**
(43) Date of publication of application: **27.03.2013**
(21) Application number: 12181940.3
(22) Date of filing: 28.08.2012
(51) Int. Cl.: A61B 5/00

(54) **Optical imaging probes and related methods**

(30) Priority: 31.08.2011 US 201113222514
(71) Applicant: Lightlab Imaging, Inc., Westford, MA 01886 (US)
(72) Inventor: Petroff, Christopher, Groton, MA Massachusetts MA 01450 (US); Kelly, David, Westford, MA Massachusetts MA 01886 (US)
(74) Representative: Lucey, Michael

(57) **Abstract**

Optical probe including a torque wire (34), an optical fiber (10) positioned within the torque wire, a beam director (18) positioned coaxial with and adjacent to one end of the optical fiber and an overcladding (26), positioned adjacent to and over the optical fiber and the beam director, the overcladding defining an air gap (30) adjacent the beam director so as to cause total internal reflection of light passing from the optical fiber through the beam director. During manufacturing, the overcladding is selectively heated to eliminate any air gap along the involved length (I_{L}) only to reduce the likelihood of brakeage or snapping of the elements in the optical train as a result of a mismatch or differences between the coefficient of thermal expansion of the overcladding and one of the optical elements, such as the beam director.

## Description

### FIELD OF THE INVENTION

The invention relates generally to imaging probes and more specifically to imaging probes for use with Optical Coherence Tomography.

### BACKGROUND

The use of a very small lens in an OCT imaging catheter allows the crossing profile of the imaging catheter to be small. This improves the ability of the catheter to reach lesions in stenotic arteries. A torque wire design and a lens design must be small enough to maintain a low crossing profile as it passes through a stenosed portion of a vessel and yet be robust enough for high-speed imaging and pullback.

The present invention addresses this need and others.

### SUMMARY

In part, the invention relates to imaging probes and components or subsystems thereof. In one embodiment, the invention relates to a probe suitable for transmitting imaging light to a lumen and collecting imaging light scattered from structures in the lumen such as the lumen wall and other structures. In one embodiment, an optical element such as a cap or overcladding is a component of the probe. The optical element can be formed from a unitary structure that includes a lens material such as doped silica glass, borosilicate glass, or a glass having a melting point lower than that of silica glass. In one embodiment, the optical element includes a bulb portion and an elongate tubular portion. The tubular portion partially defines a cavity such as an air space that allows for total internal reflection such that imaging light received through the bulb portion can be directed to an optical coherence tomography probe or other data collection system. A torque wire is configured in one embodiment to rotate the cap or overcladding and a beam director disposed therein. This enables OCT data to be collected with respect to the walls of a lumen.

In one embodiment, the invention relates to an optical probe including a torque wire; an optical fiber positioned within the torque wire; an angled fiber (or beam director) positioned coaxial with and adjacent to one end of the optical fiber; and an overcladding, positioned adjacent to and over the optical fiber and the angled fiber, the overcladding defining an air gap adjacent the angled fiber so as to cause total internal reflection of light passing from the optical fiber through the angled fiber. In one embodiment, the optical probe includes a beam expander and a beam shaper coaxial with and located between the optical fiber and the angled fiber. In another embodiment, the optical probe further includes a marker band positioned over a portion of the overcladding. In yet another embodiment, the overcladding is made of doped silica glass.

In one embodiment, an overcladding or cap surrounds a substantially linear arrangement of a first optical element, a second optical element, a third optical element and a first cavity and wherein the overcladding or cap is in communication with a second cavity having a substantially cylindrical shape, or curved shape and defined in part by a torque wire terminus and a epoxy shell having an optical fiber passing through the shell. In one embodiment, the first optical element, the second optical element and the third optical element are selected from the group consisting of an optical fiber segment, a doped optical fiber segment, a beam shaper, a beam expander, an angled polished fiber segment and a beam director.

In one embodiment, the overcladding or cap has a first coefficient of thermal expansion and one or more of the beam director, such as an angle polished fiber, the beam expander, or the beam shaper has a second coefficient of thermal expansion that differs from or is the same as the first coefficient of thermal expansion. When the coefficients of thermal expansion of the overcladding and the other optical elements differ, breakage and damage to the probe can occur during use as a result of a thermal mismatch between components. This mismatch is necessary under some circumstances to maintain other parameter relationships such as melting point differences and matched indices of refraction between the overcladding and other components. Selective coupling of thermal mismatched materials over a smaller surface area allows thermally dissimilar materials to be used in some embodiments.

In one embodiment, the beam director and the overcladding or cap have different coefficients of thermal expansion, but the same or substantially the same indexes of refraction. Further, in one embodiment, the cylindrical region of the overcladding that receives light from the beam director is selectively heated to melt and couple or bond the overcladding to the beam director while an air gap or cavity is present or defined between the interface between the beam shaper and the overcladding. In one embodiment, an air gap or cavity is present or defined between the interface between the beam expander and the overcladding. In one embodiment, the overcladding having an optical fiber disposed therein and/or coupled thereto is configured to rotate at a frequency greater than about 100 Hz. Similarly, the optical fiber that bears the tensile load and pulls the torque wire is configured to withstand bending stress and hoop stress during a pullback speed of greater than about 20 mm/s.

This Summary is provided merely to introduce certain concepts and not to identify any key or essential features of the claimed subject matter.

### BRIEF DESCRIPTION OF DRAWINGS

The figures are not necessarily to scale, emphasis instead generally being placed upon illustrative principles. The figures are to be considered illustrative in all aspects and are not intended to limit the invention, the scope of which is defined only by the claims.
Fig. 1A is a schematic longitudinal cross-section of an end of an optical imaging probe according to an illustrative embodiment of the invention;
Fig. 1B is a schematic longitudinal cross-section of an end of another optical imaging probe according to an illustrative embodiment of the invention;
Figs. 2A and 2B are a series of beam profiles as generated by an optical imaging probe having a non-overclad design such as a potted lens design and by an optical probe having an overclad design as shown in Fig. 1B, respectively; and
Fig. 3 is an exemplary process flow emphasizing certain methods steps performed when making one embodiment of an overcladding in accordance with an illustrative embodiment of the invention.

### DETAILED DESCRIPTION

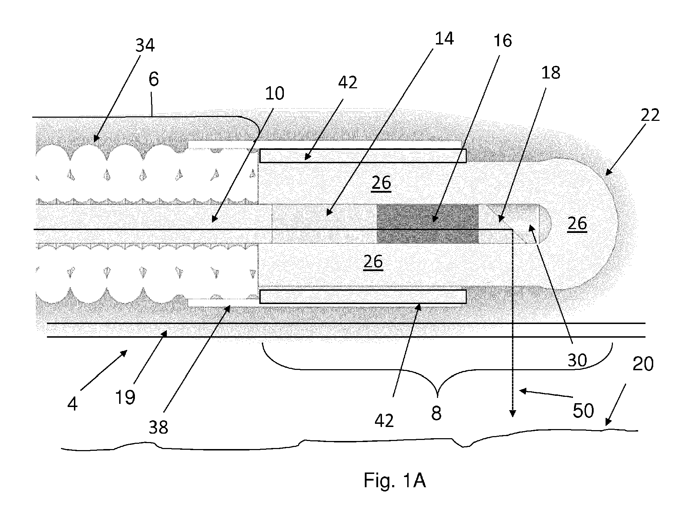

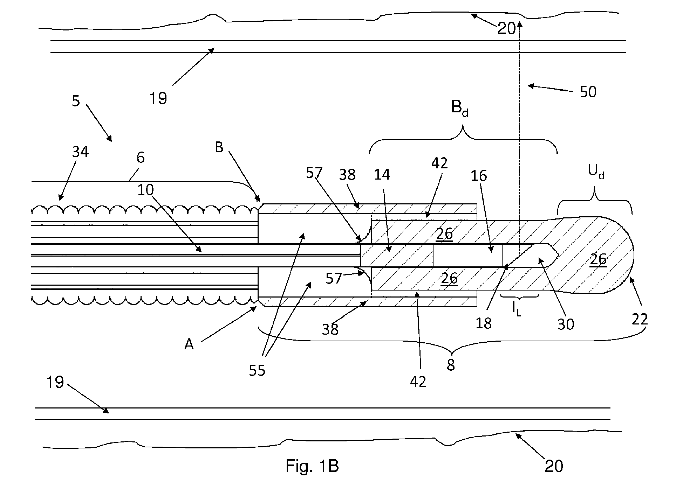

An embodiment of an optical probe 4 suitable for use in optical coherence tomography (OCT) of a vessel is shown in Fig. 1A. Another embodiment of a probe that has some additional features relative to that shown in Fig. 1A is shown in Fig. 1B as probe 5. In brief overview, the optical probe 4, 5 includes a torque wire portion 6 which includes a torque wire 34 and an optical head portion 8, which includes a single mode optical fiber 10, a beam expander 14, a beam shaper 16 and an angle polished optical fiber 18 (alternatively referred to as a beam director 18). A closed end 22 bulbous overcladding 26 (or cap) creates an air space 30 that causes total internal reflection of light by the angle polished fiber (beam director) 18 at the interface between the air space 30 and the angle polished fiber or beam director 18.

In use, a given probe embodiment 4, 5 is introduced into a catheter sheath and is moved to the position of interest in the blood vessel. Light passing through the fiber 10 is totally internally reflected and exits the angle polished fiber 18 and passes through the sheath wall 19 to the wall of the vessel 20. Light 50 passes through the single mode fiber 10 and is expanded and shaped before being reflected through the side of the probe 4, 5 by the angle polished fiber 18.

The overcladding or cap 26 can be fabricated using various techniques. The overcladding can have various shapes such as elongate, tubular, and cylindrical. The overcladding 26 is a unitary material that may be doped or undoped in various embodiments. In one embodiment, the overcladding 26 is created using a melt process with a bulb-shaped end 22. Compared to a sharp-edged tip, this smooth bulb shaped end 22 allows for the easy advancement of the optical assembly through the sheath with a reduced risk of puncturing the sheath should the sheath be kinked. During assembly, the tubular overcladding 26 is slid over the single mode fiber 10 and optical fiber segments 14, 16, and 18. The combination is then heated to form a bond and eliminate any air space between the overcladding 26 and the angle polished fiber 18. This is done because an air space between the overcladding 26 and the angled fiber 18 would disturb the light path.

In a preferred embodiment, the overcladding 26 is made from a glass or plastic that has a lower melting temperature than the angled fiber 18. In one embodiment, the overcladding includes a borosilica capillary tube. In another embodiment, the overcladding is made of a silica glass material doped or modified to lower its melting temperature relative to that of the unmodified glass material. Thus, the overcladding can be attached or coupled to the beam director or other silica-based components of the probe by selectively applying heat that will not melt the components to which it is being attached or coupled. The overcladding shown in Figs. 1A and 1B can have any suitable geometric shape suitable for introduction in a lumen. As shown in Fig. 1B, the overcladding 26 defines a bore in which optical elements are disposed. In one embodiment, the bore has a length B_{d} and the terminal end face surface has a length U_{d}.

In another embodiment, silica doped with Fluorine, to lower the silica's melting temperature, is used to make the overcladding 26. In one embodiment, the silica is doped with Fluorine at about 10% ± or 5% to 10% by weight. The lower melting temperature allows for attachment of the overcladding 26 at a lower temperature which is less likely to damage the angle polished fiber 18. The silica has a better match to the thermal coefficient of expansion of the components of the beam director or other optical elements such as the beam expander and beam shaper. This lowers various stresses and forces that can result from thermal expansion or contraction of one or more of the optical elements in the probe 4, 5.

Substantially all of the tensile force is supplied by the optical fiber. The optical fiber 10 transmits force to the torque wire 34 and pulls it along during an OCT pullback as part of an OCT imaging data collection session. The torque wire 34 applies the twisting force that rotates the optical fiber 10 and overcladding 26 and thus the beam director 18. Various stresses such as hoop stresses can be applied to the optical fiber and other epoxied or butt-coupled elements shown in Fig. 1A and 1B. However, selective application of heat, reduction of air gaps between the overcladding and the beam director 18, and efforts to match thermal coefficients of expansion and index of refraction can be used to improve the stability of a probe such that it resists breakage while also improving its optical and data collection properties.

In one embodiment, the outer diameter of the overcladding 26 is sized such that it is close to that of the torque wire 34 and is significantly larger than the fiber 10. This has two advantages. First, it reduces the possibility that bubbles are trapped on the outer surface of the overcladding 26 as it is pulled back out of the vessel during an OCT imaging scan. Such bubbles would preferentially attach if the diameter of the overcladding 26 were smaller than the torque wire 34. Second, the outer surface of the overcladding 26 allows the light to exit from the overcladding 26 at a larger diameter than the fiber 10.

In one embodiment, the marker band 38 is attached to the torque wire 34 by welding, brazing or gluing the torque wire 34 to a marker band 38. As shown in Fig. 1A, the beam expander 14, the beam shaper 16 and the angled fiber 18 with overcladding 26 is then slid into the marker band 38 and the overcladding 26 is attached or fixed with glue 42 or heat formed in place. As a result, the marker band 38 is affixed and positioned close to the angled fiber 18. This simplifies locating of the angled fiber 18 under angiography. By placing the marker band 38 at a position on the probe within the sheath, as opposed to having the marker band 38 being located on the sheath, any inaccuracies in placement of the angled fiber 18 due to the sheath stretching are avoided. The marker band 38 helps track the probe during various procedures and imaging events, such as during angiographic imaging.

By choosing a low melting point glass for the overcladding 26 the device avoids distorting the graded index profile of the fused silica of the internal micro-lens or beam directing system that includes a beam expander 14, a beam shaper 16, and a beam director 18. The index of refraction of the overcladding glass is matched with index of refraction of the beam director 18. Further, the bond/melt region of the overcladding is localized to a (small) optically active area at the angle polished fiber to minimize the thermal stresses.

In one embodiment, this optically active area is the involved length or a subset thereof. Due to a better match of the thermal coefficient of expansion, the use of a doped silica cap allows a melt bond to be formed between the cap and the beam director (such as for example an angle polished fiber) with a much lower assembled stress than a borosilicate cap. In one embodiment, the overcladding glass may be glued to the angle polished fiber. By gluing the overcladding to the angle polished fiber, mechanical stress is reduced in the assembled probe. Alternatively the overcladding glass may be formed from a capillary tube disposed on top of the angle polished fiber.

Fig. 1B is another probe embodiment that includes a modified geometry relative to that of Fig. 1A as well as other features. As shown in Fig. 1B, a probe 5 is depicted. The probe 5 includes an optical fiber suitable for transmitting light into a lumen of interest and collecting light scattered from the walls which define the lumen and other structures of interest such as plaques, lesions, or other regions of interest. A cap or overcladding 26 is a component of the probe 5 in one embodiment. As shown in Fig. 1B, a cavity 55 is defined by the surfaces or boundaries of an optical fiber 10, a portion of a torque wire 6, layers of glue 42, a marker band 38, and an epoxy material 57. In one embodiment, cavity 55 is empty. As shown, a cavity 30 that typically includes a gas or a fluid is at one end of the probe and within the overcladding 26, while cavity 55, which also typically includes a gas or a fluid, surrounds the optical fiber at another end of the probe 5.

A beam director 18, which can include an angle polished optical fiber, in one embodiment directs light into the lumen and toward the walls of the lumen. In one embodiment, the beam director 18 rotates and thus scans a circular or spiral pattern along the walls of the lumen as the probe is pulled through the lumen. The epoxy 57 shown forms a symmetric or asymmetric shape that surrounds the optical fiber 10 in one embodiment.

In one embodiment, the distance substantially along the length of the overcladding 22 over which light is received and transmitted from the beam director 18 is referred to as the involved length I_{L}. As shown, the involved length I_{L} can include a distance that includes the length of the beam director 18 or some length substantially greater than or less than this length. In one embodiment, the overcladding 26 is selectively heated such that along the involved length I_{L} there is substantially no air gaps between the overcladding and the involved length I_{L}. In one embodiment, air gaps are defined by regions outside of the involved length along the interface between the overcladding 26 and optical elements coaxial with the fiber 10.

An epoxy material 57 is applied at one end face of the overcladding as shown. In one embodiment, the epoxy material 57 also contacts the optical fiber 10 and the beam expander 14. The epoxy material provides strain relief for optical fiber 10. In one embodiment, the epoxy forms a frustum or a frustoconical like shape with one surface contacting the overcladding 26 and one surface contacting the beam expander 14 and the fiber 10. As shown, the beam expander 14 protrudes past the substantially flat end-face of the overcladding 26 and is butt-coupled to fiber 10 in one embodiment.

As shown, in Fig. 1B, the optical fiber 10 is coupled to a beam expander 14 at the two end faces as shown. Further, the beam expander 14 is coupled to a beam shaper 16 as shown. Beam director 18 is likewise coupled to the beam shaper 16. Each of these elements may be coupled by butt-coupling or splicing fiber segments in one embodiment. As shown, in Fig. 1B, the marker band 38 is welded or otherwise attached to the torque wire 34 such as for example at positions A and B as shown at the junction of the torque wire 34 and the marker band 38.

The overcladding or cap 26 can be fabricated using various techniques. In one embodiment, the overcladding 26 has a substantially tubular geometry with a flared or bulbous end. The overcladding 26 can be created using a melt process with a bulb-shaped end 22. Compared to a sharp-edged tip, this smooth bulb shape 22 allows for the easy advancement of the optical assembly through the sheath with a reduced risk of puncturing the sheath should the sheath be kinked.

In one embodiment, this elimination or substantial reduction in any air gap is only performed along the involved length to reduce the likelihood of breakage or snapping of the elements in the optical train as a result of a mismatch or differences between the coefficient of thermal expansion of the overcladding and one of the optical elements, such as for example, the beam director 18.

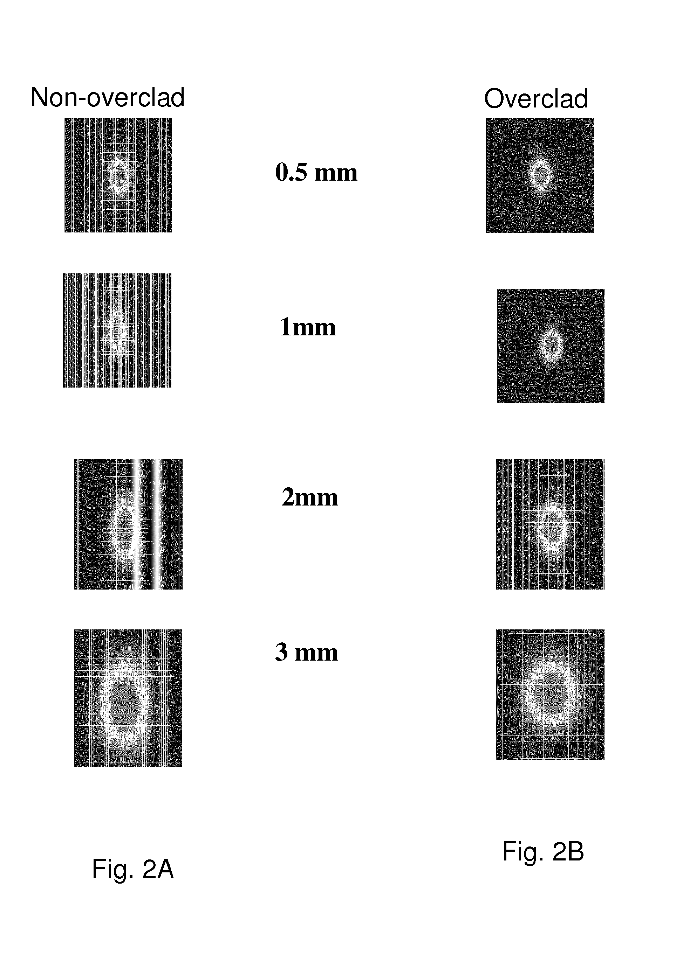

Referring to Fig. 2A and 2B, the beam profile images show multiple beam profile at different distances from the glass overcladding 26. Tables 1 and 2 included below list the intensities of the beam taken across the beam profiles. In the glass overclad design at around 1 mm from the glass overcladding, the beam is at the sharpest focus. The full width half-maximum (FWHM) beam size is 20 microns in x direction (The plane parallel to catheter axis.) and 40 microns in y direction (The plane perpendicular to the catheter axis). In general, the smaller the FWHM, the better. If the FWHM is large it is too difficult to identify individual features in the image. The FWHM is measured in two planes because there is cylindrical distortion only in Y plane. A comparison of the standard flat probe end design with the current glass overcladding shows that the FWHM in Y is much smaller. This means the beam is better focused around the circumference.

**Table 1 - Non-overclad Embodiment**

| **WD [mm]** | **FWH M-X [um]** | **FWH M-Y [um]** | **Fiber Coupling** |
|---|---|---|---|
| 0.5 | 24 | 38 | 38% |
| 1 | 20 | 40 | 39% |
| 2 | 26 | 46 | 37% |
| 3 | 42 | 56 | 24% |

**Table 2 -Overclad Embodiment**

| **WD [mm]** | **FWH M-X [um]** | **FWH M-Y [um]** | **Fiber Coupling** |
|---|---|---|---|
| 0.5 | 24 | 46 | 28% |
| 1 | 20 | 53 | 29% |
| 2 | 27 | 65 | 20% |
| 3 | 42 | 78 | 13% |

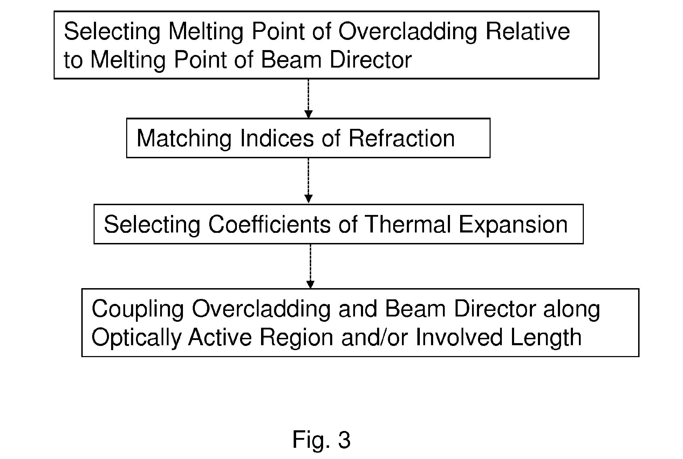

Fig. 3 is a method of fabricating a probe according to one embodiment of the invention. In Fig. 3, various methods steps performed when designing or fabricating an optical imaging probe having an overcladding are described. As shown, one step is selecting the melting point of the overcladding relative to the melting point of beam director. This allows one or more surfaces of the overcladding to be melted or shrunk by selectively applying heat such that the overcladding couples to the beam director without air gaps there between. The optical indices of refraction for the beam director and the overcladding are also matched.

Unfortunately, matching each index of refraction for the overcladding and the beam director while controlling for the relationship of the melting points described above results in other challenges. Specifically, these constraints results in challenges in also matching the coefficients of thermal expansion for the overcladding and beam director. In various embodiments, the coefficients of thermal expansion cannot be matched and also satisfy the other constraints relating to melting point and index of refraction. As a result, these can be chosen to be different in one embodiment. However, air gaps need to be reduced between the beam director and overcladding. Also, the surface area over which the overcladding and elements coaxial with the optical fiber are coupled need to be reduced to prevent snapping or breaking due to variations in the coefficients of thermal expansion. This last issue can be mitigated by coupling overcladding and beam director along optically active region and/or involved length.

The aspects, embodiments, features, and examples of the invention are to be considered illustrative in all respects and are not intended to limit the invention, the scope of which is defined only by the claims. Other embodiments, modifications, and usages will be apparent to those skilled in the art without departing from the spirit and scope of the claimed invention.

The use of headings and sections in the application is not meant to limit the invention; each section can apply to any aspect, embodiment, or feature of the invention.

Throughout the application, where compositions are described as having, including, or comprising specific components, or where processes are described as having, including or comprising specific process steps, it is contemplated that compositions of the present teachings also consist essentially of, or consist of, the recited components, and that the processes of the present teachings also consist essentially of, or consist of, the recited process steps.

In the application, where an element or component is said to be included in and/or selected from a list of recited elements or components, it should be understood that the element or component can be any one of the recited elements or components and can be selected from a group consisting of two or more of the recited elements or components. Further, it should be understood that elements and/or features of a composition, an apparatus, or a method described herein can be combined in a variety of ways without departing from the spirit and scope of the present teachings, whether explicit or implicit herein.

The use of the terms "include," "includes," "including," "have," "has," or "having" should be generally understood as open-ended and non-limiting unless specifically stated otherwise.

The use of the singular herein includes the plural (and vice versa) unless specifically stated otherwise. Moreover, the singular forms "a," "an," and "the" include plural forms unless the context clearly dictates otherwise. In addition, where the use of the term "about" is before a quantitative value, the present teachings also include the specific quantitative value itself, unless specifically stated otherwise.

It should be understood that the order of steps or order for performing certain actions is immaterial so long as the present teachings remain operable. Moreover, two or more steps or actions may be conducted simultaneously.

Where a range or list of values is provided, each intervening value between the upper and lower limits of that range or list of values is individually contemplated and is encompassed within the invention as if each value were specifically enumerated herein. In addition, smaller ranges between and including the upper and lower limits of a given range are contemplated and encompassed within the invention. The listing of exemplary values or ranges is not a disclaimer of other values or ranges between and including the upper and lower limits of a given range.

The invention is not limited to the embodiments hereinbefore described but may be varied in both construction and detail.

## Claims

1. An optical probe comprising:
a torque wire configured to rotate;
an optical fiber positioned within the torque wire;
a beam director positioned coaxial with and adjacent to one end of the optical fiber and having a first coefficient of thermal expansion; and
an overcladding, positioned adjacent to and over the optical fiber and the beam director, the overcladding defining an air gap adjacent the beam director so as to cause total internal reflection of light passing from the optical fiber through the beam director, the overcladding having a second coefficient of thermal expansion, wherein the beam director and the overcladding are coupled along an involved length that is substantially free of any gaps and wherein the interface between the overcladding and the beam director outside of the involved length defines one or more gaps configured to reduce stress on the optical probe.

2. The optical probe of claim 1 further comprising a beam expander and a beam shaper coaxial with and located between the optical fiber and the beam director.

3. The optical probe of claim 1 or 2 further comprising a marker band positioned over a portion of the overcladding.

4. The optical probe of any preceding claim wherein the first coefficient of thermal expansion and the second coefficient of thermal expansion differ.

5. An optical coherence tomography probe cap configured to transmit and receive light comprising:
an elongate unitary member having a first end and a second end and a longitudinal axis, the elongate unitary member defining a bore having a bore diameter, wherein the second end comprises a terminal bulbous surface, wherein the elongate unitary member is substantially cylindrical in shape from the first end along the longitudinal axis before transitioning to the bulbous surface, wherein the elongate unitary member has a coefficient of thermal expansion that differs from the coefficient of thermal expansion of a silica optical fiber.

6. The optical coherence tomography probe cap of claim 5 wherein the bore diameter is sized to receive the silica optical fiber.

7. The optical coherence tomography probe cap of claim 5 or 6 further comprising a marker band in which the elongate unitary member is partially disposed and adhered to, the marker band partially defining a cavity for receiving the silica optical fiber.

8. The optical coherence tomography probe cap of any of claims 5 to 7 further comprising a beam director disposed within the bore such that light directed along the longitudinal axis propagates from the beam director at an angle substantially normal to the longitudinal axis.

9. The optical coherence tomography probe cap of any of claims 5 to 8 wherein the elongate unitary member comprise a material selected from the group consisting of glass, plastic, doped glass, and Fluorine doped glass, Boron doped glass, and a polymer.

10. The optical coherence tomography probe cap of claim 8 further comprising an air filled cavity defined by both the bore and the beam director.

11. The optical coherence tomography probe cap of claim 8 or 9 further comprising a first optical fiber portion in optical communication with the beam director and at least partially disposed within the bore.

12. The optical coherence tomography probe cap of claim 11 further comprising a marker band in which the elongate unitary member is partially disposed in and adhered to, the marker band partially defining a cavity configured to receive the first optical fiber portion.

13. The optical coherence tomography probe cap of claim 9 wherein the air filled cavity is positioned to cause total internal reflection of light at an interface between the beam director and the air filled cavity.

14. A method of making a cap comprising a first material and configured to receive an optical assembly comprising a second material and configured to collect imaging data comprising:
(a) selecting the first material such that it has a first melting point that is less than a second melting point of the second material;
(b) matching a first index of refraction of the first material with a second index of refraction of the second material;
(c) mismatching a first coefficient of thermal expansion of the first material relative to a second coefficient of thermal expansion of the second material to satisfy steps (a) and (b);
(d) melting the first material such that it couples with the second material along an involved length of the optical assembly such that gaps remain along an interface between the first material and the second material beyond the involved length.

15. The method of claim 14 further comprising the step of coupling a torque wire to the cap and disposing an optical fiber therein, wherein the optical fiber is a component of the optical assembly and/or
further comprising the step of doping the first material to change its melting point and/or
further comprising the step of applying epoxy around a region of the optical fiber to reduce one or more forces applied thereto.
